# EUROPEAN PATENT APPLICATION

(11) **EP 4 759 931 A1**
(43) Date of publication of application: **17.06.2026**
(21) Application number: 24854126.0
(22) Date of filing: 30.07.2024
(51) Int. Cl.: C12N 15/31, C12N 1/21, C12N 15/63, C12P 21/02

(54) **SECRETION SIGNAL PEPTIDE GENE DERIVED FROM BACTERIUM OF GENUS HYDROGENOPHILUS**

(30) Priority: 11.08.2023 JP 2023131718
(71) Applicant: Utilization of Carbon Dioxide Institute Co.,Ltd., Tokyo 135-0091 (JP)
(72) Inventor: YUKAWA Hideaki, Tokyo 135-0091 (JP)
(74) Representative: Vossius & Partner Patentanwälte Rechtsanwälte mbB
(86) International application number: PCT/JP2024/027180
(87) International publication number: WO 2025/037535

(57) **Abstract**

The DNA of the following (a), (b), or (c) is novel secretory signal peptide gene which can be used in secretory production of proteins by microorganisms.
(a) a DNA encoding a peptide comprising the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151.
(b) a DNA encoding a peptide comprising an amino acid sequence that has 90% or more identity with the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.
(c) a DNA encoding a peptide comprising an amino acid sequence that has 1 to 5 amino acids deleted, substituted, inserted, or added in the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.

## Description

### Technical Field

The present invention relates to novel secretory signal peptide genes, a transformant or recombinant obtained by introduction of any of the secretory signal peptide genes, and a method for producing a target protein using the transformant or recombinant.

### Background Art

Microbial protein production through genetic engineering techniques has used various hosts such as *Escherichia coli,* yeast, and *Bacillus subtilis.* Protein production using these hosts relies on sugars derived from biomass as a carbon source. However, the use of biomass requires complex processes for converting biomass into sugars and thus is costly, and the industrial use of biomass can have adverse environmental impacts despite its intended purpose.

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

As part of research on decarbonization, gases such as carbon dioxide, methane, and carbon monoxide have attracted attention as more sustainable carbon sources, and techniques for producing valuable substances using microorganisms that utilize these gases are the subject of interest. In particular, carbon fixation and efficient utilization of carbon dioxide, a significant contributor to global warming, is highly anticipated. Material production through carbon dioxide fixation avoids the drawbacks of methods using sugars derived from biomass as a carbon source.

Hydrogen-oxidizing bacteria or hydrogen bacteria, can grow using the chemical energy generated from the reaction of hydrogen and oxygen as well as using carbon dioxide as their sole carbon source. These bacteria can produce chemical products from a mixture of oxygen, hydrogen, and carbon dioxide gases as source materials. Therefore, the bacteria can efficiently convert carbon dioxide into organic compounds and can be cultured in a simple culture medium. Hydrogen-oxidizing bacteria typically grow slowly, but among them, *Hydrogenophilus* bacteria grow at a remarkably faster rate. The Journal of Mitsubishi Research Institute, No. 34, 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms". As described above, *Hydrogenophilus* bacteria have an atypically remarkable carbon dioxide fixation ability among organisms having carbon dioxide fixation ability; this has led to a desire to use *Hydrogenophilus* bacteria as hosts for material production.

In microbial protein production, secretory production of proteins offers advantages such as very easy purification of the produced proteins. This has driven numerous efforts to achieve secretory production of proteins by microorganisms. Previous reports include techniques for secretory production of proteins by *Bacillus* bacteria (Non Patent Literature 1), a methylotrophic yeast, *Pichia pastoris* (Non Patent Literature 2), a coryneform bacterium, *Corynebacterium glutamicum* (Patent Literature 1), and *Aspergillus* filamentous fungi (Non Patent Literature 3). However, there have been no reports on techniques for secretory production of proteins by hydrogen-oxidizing bacteria including *Hydrogenophilus* bacteria.

For extracellular secretion of proteins, they first need to pass through the cell membrane via a transmembrane assembly (translocon) on the cell membrane. The translocon recognizes secretory proteins on the basis of the presence of a secretory signal peptide at the N-terminus of the proteins, which plays an important role in such recognition. Typical secretory signal peptides of microorganisms have a length of about 20 amino acids in which many basic amino acids near the N-terminus are followed by consecutive hydrophobic amino acids. The secretory signal peptide is ultimately cleaved by a signal peptidase on the cell membrane. The cleavage site, i.e., the C-terminus of the secretory signal peptide, usually contains low-molecular-weight amino acids. Known examples of the membrane translocation include one in which translocation is coupled with protein synthesis on a ribosome bound to a translocon; and another in which precursor protein synthesis in the cytoplasm is followed by translocation into the periplasm via a translocon to which a Sec complex has been attached. Thus, a number of proteins are involved in the secretion of a single protein. The structure of the secretory signal peptide affects the selection of the secretion pathway for secretory proteins, resulting in significant impacts on the secretion efficiency of secretory proteins.

### Citation List

### Patent Literature

[Patent Literature 1] U.S. Patent No. 4965197

### Non Patent Literature

[Non Patent Literature 1] Microbiology Reviews, 57, 109-137 (1993)
[Non Patent Literature 2] Bio/technology, 11, 905-910 (1993)
[Non Patent Literature 3] Bio/technology, 6, 1419-1422 (1988)
[Non Patent Literature 4] Journal of Molecular Biology, 362, 393-402 (2006)

### Summary of Invention

### Technical Problem

An objective of the present invention is to provide a novel secretory signal peptide gene that can be used for secretory production of proteins by microorganisms. In particular, an objective of the present invention is to provide a novel secretory signal peptide gene that can be used for secretory production of proteins by *Hydrogenophilus* bacteria.

### Solution to Problem

As described in the section "EXAMPLES", the present inventor analyzed proteins contained in the culture supernatant of *Hydrogenophilus thermoluteolus,* performed *in silico* searches and functional predictions based on these protein data, selected proteins containing a secretory signal sequence in their N-terminal region from the analyzed proteins, and finally found a number of secretory signal peptides encoded in the genome of *Hydrogenophilus thermoluteolus.*

The present invention has been completed on the basis of the above findings and provides the following [1] to [11].
[1] A DNA of the following (a), (b), or (c).
   (a) a DNA encoding a peptide comprising the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151.
   (b) a DNA encoding a peptide comprising an amino acid sequence that has 90% or more identity with the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.
   (c) a DNA encoding a peptide comprising an amino acid sequence that has 1 to 5 amino acids deleted, substituted, inserted, or added in the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.
[2] The DNA according to [1], wherein the DNA is a DNA of the following (d) or (e).
   (d) a DNA comprising the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152.
   (e) a DNA comprising a nucleotide sequence that has 90% or more identity with the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152 and encoding a peptide functioning as a secretory signal peptide.
[3] A secretory signal peptide gene comprising the DNA according to [1] or [2].
[4] A vector comprising the DNA according to [1] or [2].
[5] A vector for expressing a target protein, the vector comprising the gene of the target protein downstream of the DNA according to [1] or [2].
[6] A transformant obtained by introducing, into a host microorganism, a DNA fragment containing the DNA according to [1] or [2] and the structural gene of a target protein located downstream of the DNA, or the vector according to [5].
[7] The transformant according to [6], wherein the host microorganism is a *Hydrogenophilus* bacterium.
[8] A method for producing a target protein, the method comprising the steps of: culturing the transformant according to [7] using substantially only carbon dioxide as a carbon source; and collecting the protein from culture medium.
[9] A recombinant obtained by introducing the DNA according to [1] or [2] into the upstream of the structural gene of a target protein in the genome of a microorganism.
[10] The recombinant according to [9], wherein the microorganism is a *Hydrogenophilus* bacterium.
[11] A method for producing a target protein, the method comprising the steps of: culturing the recombinant according to [10] using substantially only carbon dioxide as a carbon source; and collecting the protein from culture medium.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing organisms allows the production of organic substances that can be used as food, feed, fuel, or the like and the production of raw materials of chemical products. In other words, carbon dioxide itself can be used as resources and directly converted into valuable substances. Accordingly, the twin problems of global warming due to increased atmospheric carbon dioxide and scarcity of food, feed, fuel, and raw materials of chemical products can be solved.

Hydrogen-oxidizing bacteria, which can grow using the chemical energy generated from the reaction of hydrogen and oxygen as well as using carbon dioxide as their sole carbon source, can grow using a mixture of oxygen, hydrogen, and carbon dioxide gases as source materials. Therefore, the bacteria can efficiently convert carbon dioxide into organic compounds and can be cultured in a simple culture medium. Hydrogen-oxidizing bacteria typically grow slowly, but *Hydrogenophilus* bacteria grow at a remarkably faster rate. The Journal of Mitsubishi Research Institute, No. 34, 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

The secretory signal peptides of the present invention are derived from the genome of *Hydrogenophilus thermoluteolus,* but are thought to be capable of functioning in a broad range of microorganisms such as bacteria, and particularly functions efficiently in *Hydrogenophilus* bacteria, more particularly *Hydrogenophilus thermoluteolus.*

When a DNA fragment containing a secretory signal peptide gene of the present invention joined upstream of the gene of a target protein is introduced into a *Hydrogenophilus* bacterium, the resulting transformant produces the target protein as a fusion product with the secretory signal peptide during culture. Through subsequent processing, a mature form of the protein, which is free from the secretory signal peptide, is secreted from the cell. Thus, the present invention enables the accumulation of the target protein in culture medium, leading to easy collection of the protein. Furthermore, it is easier to purify the target protein because smaller amounts of impurities are present than in cases where the target protein is collected after cell disruption. Therefore, efficient protein production can be achieved.

As described above, *Hydrogenophilus* bacteria grow very fast using carbon dioxide as their sole carbon source; therefore, when these bacteria are modified using a secretory signal peptide gene of the present invention to extracellularly produce a target protein, very efficient production of the target protein can be achieved.

Previous studies on protein secretion using *Bacillus* bacteria revealed that the secretory signal sequence enabling efficient secretion of a protein to be expressed varies considerably depending on the protein and that it is desirable to optimize the secretory signal sequence for each individual protein intended for secretory production (Non Patent Literature 4).

In this regard, the present invention provides as many as 76 secretory signal peptides that function efficiently in *Hydrogenophilus* bacteria, and from these secretory signal peptides, a suitable one for secretory production of a target protein can be easily selected.

### Brief Description of Drawings

[Fig. 1] Fig. 1 shows the matching results between the amino acid sequences of predicted secretory proteins from *Hydrogenophilus thermoluteolus* and the amino acid sequences of peptides detected by mass spectrometry.
[Fig. 2] Fig. 2 shows the matching results between the amino acid sequences of predicted secretory proteins from *Hydrogenophilus thermoluteolus* and the amino acid sequences of peptides detected by mass spectrometry.
[Fig. 3] Fig. 3 shows the matching results between the amino acid sequences of predicted secretory proteins from *Hydrogenophilus thermoluteolus* and the amino acid sequences of peptides detected by mass spectrometry.
[Fig. 4] Fig. 4 shows the matching results between the amino acid sequences of predicted secretory proteins from *Hydrogenophilus thermoluteolus* and the amino acid sequences of peptides detected by mass spectrometry.
[Fig. 5] Fig. 5 shows the matching results between the amino acid sequences of predicted secretory proteins from *Hydrogenophilus thermoluteolus* and the amino acid sequences of peptides detected by mass spectrometry.
[Fig. 6] Fig. 6 shows the matching results between the amino acid sequences of predicted secretory proteins from *Hydrogenophilus thermoluteolus* and the amino acid sequences of peptides detected by mass spectrometry.
[Fig. 7] Fig. 7 shows the matching results between the amino acid sequences of predicted secretory proteins from *Hydrogenophilus thermoluteolus* and the amino acid sequences of peptides detected by mass spectrometry.
[Fig. 8] Fig. 8 shows the matching results between the amino acid sequences of predicted secretory proteins from *Hydrogenophilus thermoluteolus* and the amino acid sequences of peptides detected by mass spectrometry.
[Fig. 9] Fig. 9 shows the matching results between the amino acid sequences of predicted secretory proteins from *Hydrogenophilus thermoluteolus* and the amino acid sequences of peptides detected by mass spectrometry.
[Fig. 10] Fig. 10 shows the matching results between the amino acid sequences of predicted secretory proteins from *Hydrogenophilus thermoluteolus* and the amino acid sequences of peptides detected by mass spectrometry.
[Fig. 11] Fig. 11 shows the matching results between the amino acid sequences of predicted secretory proteins from *Hydrogenophilus thermoluteolus* and the amino acid sequences of peptides detected by mass spectrometry.
[Fig. 12] Fig. 12 shows the matching results between the amino acid sequences of predicted secretory proteins from *Hydrogenophilus thermoluteolus* and the amino acid sequences of peptides detected by mass spectrometry.

### Description of Embodiments

Hereinafter, the present invention will be described in detail.

### (1) Secretory signal peptide genes

The present invention provides a DNA encoding a peptide comprising the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151, in particular, a DNA encoding a peptide consisting of the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151.

Representative examples of these DNAs include a DNA comprising the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152, in particular, a DNA consisting of the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152.

The present invention also provides a DNA encoding a peptide comprising an amino acid sequence that has 90% or more, particularly 95% or more, particularly 98% or more, particularly 99% or more identity with the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide, in particular, a DNA encoding a peptide consisting of an amino acid sequence that has 90% or more, particularly 95% or more, particularly 98% or more, particularly 99% or more identity with the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.

The present invention further provides a DNA encoding a peptide comprising an amino acid sequence that has 1 to 5, 1 to 4, 1 to 3, or 1 to 2 amino acids or 1 amino acid deleted, substituted, inserted, or added in the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide, in particular, a DNA encoding a peptide consisting of an amino acid sequence that has 1 to 5, 1 to 4, 1 to 3, or 1 to 2 amino acids or 1 amino acid deleted, substituted, inserted, or added in the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.

A particularly preferable mutation is amino acid substitution, in particular, a single amino acid substitution. The substitution of an amino acid residue is preferably conservative substitution, i.e., a substitution between amino acids having similar properties. Preferable examples of the conservative substitution include substitutions between Val, Ile, Leu, Ala, and Met; substitutions between Asp and Glu; substitutions between Asn and Gln; substitutions between Ser, Thr, Gly, and Ala; substitutions between Lys, Arg, and His; and substitutions between Phe, Tyr, and Trp. Among them, substitutions between Gly and Ala; substitutions between Val, Ile, and Leu; substitutions between Asp and Glu; substitutions between Asn and Gln; substitutions between Ser and Thr; substitutions between Lys and Arg; and substitutions between Phe and Tyr are preferable.

Representative examples of homologues of these DNAs include a DNA comprising a nucleotide sequence that has 90% or more, particularly 95% or more, particularly 98% or more, particularly 99% or more identity with the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152 and encoding a peptide functioning as a secretory signal peptide, in particular, a DNA consisting of a nucleotide sequence that has 90% or more, particularly 95% or more, particularly 98% or more, particularly 99% or more identity with the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152 and encoding a peptide functioning as a secretory signal peptide.

The above-described DNAs of the present invention can be used as secretory signal peptide genes.

In the present invention, when a transformant is obtained by introducing, into *Hydrogenophilus thermoluteolus* as a host, a DNA fragment containing a test DNA joined adjacent to the 5' end of a non-secretory protein gene encoded in the genome of *Hydrogenophilus thermoluteolus* and the resulting transformant secretes a greater amount of the protein into culture supernatant during culture than the original host strain, the test DNA is determined to function as a secretory signal peptide gene. In the present invention, the non-secretory protein refers to a protein that is not predicted to be a secretory protein by either of secretory signal peptide prediction programs PrediSi and SignalP.

In view of codon degeneracy or codon preference in host microorganisms (particularly bacteria, more particularly hydrogen-oxidizing bacteria, and even more particularly *Hydrogenophilus* bacteria), the DNA sequence encoding a protein comprising the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 may be subjected to a variety of nucleotide substitutions in the coding region, as long as such substitutions do not alter the amino acid sequence of the protein expressed from the coding region.

In the present invention, the identities of nucleotide sequences and amino acid sequences were calculated using GENETYX ver. 17 (GENETYX).

In the present invention, a DNA comprising a nucleotide sequence that has 90% or more but less than 100% identity with the nucleotide sequence of a given DNA and encoding a peptide functioning as a secretory signal peptide is called a homolog of the given DNA. A DNA encoding a peptide comprising an amino acid sequence that has 90% or more but less than 100% identity with the amino acid sequence of a peptide encoded by a given DNA and functioning as a secretory signal peptide is also called a homolog of the given DNA. A DNA encoding a peptide comprising an amino acid sequence that has 1 to 5 amino acids deleted, substituted, inserted, or added in the amino acid sequence of a peptide encoded by a given DNA and functioning as a secretory signal peptide is also called a homolog of the given DNA.

In the present invention, a peptide comprising an amino acid sequence that has 90% or more but less than 100% identity with the amino acid sequence of a given peptide and functioning as a secretory signal peptide is called a homolog of the given peptide. A peptide comprising an amino acid sequence that has 1 to 5 amino acids deleted, substituted, inserted, or added in the amino acid sequence of a given peptide and functioning as a secretory signal peptide is also called a homolog of the given peptide.

### (2) Vector

The vector of the present invention is a vector comprising the DNA of the present invention that is capable of functioning as a secretory signal peptide gene as described above.

Examples of the vector include plasmid vectors, viral vectors, cosmids, phosmids, and BACs.

The vector contains a DNA that controls the autonomous replication function in host microorganisms, particularly bacteria, and more particularly *Hydrogenophilus* bacteria. Examples of the DNA that controls the autonomous replication function in *Hydrogenophilus* bacteria include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), and vectors obtained by genetically modifying these vectors (e.g., pCAMO-6). Among them, pCAMO-6 is preferable. Those skilled in the art can prepare pCAMO-6 according to the section "EXAMPLES".

Examples of a promoter in the vector include tac promoter, lac promoter, trc promoter, and promoters OXB1 and OXB11 to OXB20 from Oxford Genetics Ltd. Examples of a terminator in the vector include T1T2 terminator of *Escherichia coli* rRNA operon rrnB, t0 transcription terminator of bacteriophage A, and T7 terminator.

The vector of the present invention contains an insertion site for the gene of a target protein downstream of the secretory signal peptide gene. The structural gene of the target protein (hereinafter sometimes referred to as "the gene of the target protein") is inserted into this gene insertion site either directly or via a plurality of nucleotides (the number of the plurality of nucleotides are a multiple of three) without altering any translation reading frame, thereby creating a vector for expressing the target protein. The resulting vector can be used for secretory production of the target protein by a host. This expression vector allows for the expression of a fusion protein of the secretory signal peptide and the target protein.

Any number of nucleotides may intervene between the secretory signal peptide gene and the gene of the target protein as long as the processing of the secretory signal peptide normally occurs, resulting in the secretion of the target protein. The number of intervening nucleotides is preferably be 300 or less, 90 or less, 30 or less, or 9 or less. For example, the intervening sequence can be a restriction enzyme recognition sequence.

The gene of the target protein may be an exogenous gene (heterologous gene), which is not present in the genome of the host microorganism, or an endogenous gene, which is present in the genome of the host microorganism. An exogenous gene (heterologous gene) is preferable.

The target protein may be a non-secretory protein or a secretory protein. When the target protein of which the gene is to be inserted into the vector of the present invention is an inherently secretory protein, a DNA encoding a portion of the target protein other than its secretory signal peptide can be used as the gene of the target protein. By expressing a fusion protein in which the portion of the target protein other than its secretory signal peptide is fused to a secretory signal peptide having higher secretion efficiency than the original secretory signal peptide, an improved secretion efficiency of the target protein is achieved.

Secretory signal peptides enabling efficient secretion of a target protein can be selected as follows. The amount of the target protein secreted into culture supernatant is compared between a transformant obtained by introducing a test vector containing a secretory signal peptide gene and the gene of the target protein downstream thereof into a host microorganism, and a transformant obtained by introducing a control vector identical to the test vector except for lacking the secretory signal peptide gene into a host microorganism of the same type. A secretory signal peptide gene which provides a greater amount of the target protein secreted may be selected.

The transformant of the present invention is a transformant obtained by introducing, into a host microorganism, a DNA fragment containing the DNA of the present invention that functions as the secretory signal peptide gene described above and a structural gene of a target protein located downstream thereof. In other words, the transformant of the present invention is a microorganism containing an exogenous DNA fragment containing the DNA of the present invention that functions as the secretory signal peptide gene described above and a structural gene of a target protein located downstream thereof.

The DNA fragment may be introduced directly into the host microorganism, or alternatively, a vector into which the DNA fragment has been incorporated (the vector of the present invention described above) may be introduced into the host microorganism.

Introduction of the DNA fragment can be carried out using conventional methods for introducing foreign genes into microorganisms, and examples thereof include calcium chloride method, rubidium chloride method, and electric pulse method (electroporation).

Examples of host microorganisms include prokaryotes such as bacteria and archaea, and eukaryotes such as fungi, plants, and algae. Among these, prokaryotes are preferred, bacteria are more preferred, hydrogen-oxidizing bacteria are still more preferred, and *Hydrogenophilus* bacteria are particularly preferred.

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus, Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable since it has top-level growth rate and carbon dioxide fixation ability among carbon dioxide fixing microorganisms.

*Hydrogenophilus* bacteria can be easily isolated from diverse regions everywhere on the earth. A preferable strains of *Hydrogenophilus thermoluteolus* include strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977)) (It proliferates double per hour.). *Hydrogenophilus thermoluteolus* strain NBRC 14978 is internationally deposited under the Budapest Treaty, and is thus publicly available.

The host microorganisms may be microorganisms isolated from nature or genetically modified microorganisms derived from microorganisms isolated from nature. Genetic modification may be intended, for example, to allow high expression of an introduced gene.

For bacteria, such a genetic modification can be made, for example, by removing (curing) endogenous plasmids in bacteria. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### (3) Recombinant

The recombinant of the present invention is a recombinant in which the DNA of the present invention that functions as a secretory signal peptide gene is inserted, directly or via a number of nucleotides (wherein the number of nucleotides is a multiple of three), into the 5'-terminal side of a structural gene of a non-secretory protein on the genome of a microorganism, or in which a secretory signal peptide gene of a secretory protein is replaced with the DNA of the present invention that functions as a secretory signal peptide gene.

The microorganism is as described above with respect to the host microorganism of the transformant of the present invention.

The number of nucleotides that can intervene between the DNA of the present invention and the structural gene of a non-secretory protein or the gene portion other than that encoding the secretory signal peptide of a secretory protein is the same as that described for the vector of the present invention.

In either case where the DNA of the present invention is introduced upstream of the structural gene of a non-secretory protein, or where the DNA of the present invention is introduced by replacement of the secretory signal peptide gene of a secretory protein, the DNA of the present invention can be introduced by homologous recombination using nucleotide sequences on both sides of the position on the genome that introduction is desired.

When the DNA of the present invention is inserted upstream of the structural gene of a non-secretory protein present on the genome, the protein becomes secreted. When the gene encoding the original secretory signal peptide of a secretory protein is replaced with the secretory signal peptide gene of the present invention, secretion efficiency of the protein can be improved by selecting, from the DNAs of the present invention, a secretory signal peptide gene suitable for secretion of the protein.

### (2) Method for producing a protein

The present invention provides a method for producing a target protein including a step of culturing the transformant or recombinant of the present invention described above, and a step of collecting the target protein from medium.

For illustrative purpose, hydrogen-oxidizing bacteria (particularly *Hydrogenophilus* bacteria) is used as an example, the transformant or recombinant may be cultured using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that the target protein can be produced efficiently.

Hydrogen-oxidizing bacteria (particularly *Hydrogenophilus* bacteria) can grow using hydrogen as a source of energy and using carbon dioxide as a sole carbon source, and thus, carbon dioxide can be fixed efficiently through the production of the target protein. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using substantially only carbon dioxide as a carbon source" and "using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and the target protein can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, shaking culture can be carried out while continuously supplying mixed gases into a culture container, or the transformant or recombinant may be cultured while introducing mixed gases into culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and target protein can be produced efficiently.

The supply rate of mixed gases or raw material gases can be 10 to 60 L/hour, preferably 10 to 40 L/hour, more preferably 10 to 20 L/hour, per 1 L of culture medium. When the supply rate is within this range, transformants or recombinants grow well and the target protein can be produced efficiently, and the amount of wasted mixed gases can be reduced.

The culture temperature is preferably 35 to 55°C, more preferably 37 to 52°C, and even more preferably 50 to 52°C. When the temperature is within this range, transformants or recombinants grow well, and the target protein can be produced efficiently.

By culturing as described above, a fusion protein of the secretory signal peptide and the target protein is produced, processed intracellularly, and the target protein is secreted extracellularly. The target protein can be recovered by collecting the culture medium, and further, the target protein can be separated and purified from the reaction solution by known methods. Examples of such known methods include methods utilizing differences in solubility, such as salting-out and solvent precipitation methods; methods utilizing differences in molecular weight, such as dialysis, ultrafiltration, and gel electrophoresis; methods utilizing differences in charge, such as ion-exchange chromatography; methods utilizing specific affinity, such as affinity chromatography; methods utilizing differences in hydrophobicity, such as reverse-phase high-performance liquid chromatography; and methods utilizing differences in isoelectric point, such as isoelectric focusing.

Although culturing using a liquid medium has been described, the transformant or recombinant of the present invention may also be cultured using a solid medium, and the target protein may be recovered from the solid medium.

Examples of methods for confirming the isolated and purified protein include Western blotting and activity assays. The structure of the purified protein can be elucidated by amino acid analysis, amino-terminal analysis, primary structure analysis, and the like.

### EXAMPLES

The present invention will now be described with reference to examples. The technical scope of the present invention is not limited by the following examples.

### (1) Identification of secretory proteins contained in culture supernatant of Hydrogenophilus bacteria (1-1) Preparation of protein samples from culture supernatant

*Hydrogenophilus thermoluteolus* strain TH-1 was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)] in a test tube. The test tube was filled with a gas mixture of H₂:O₂:CO₂ = 7.5:1:1.5, and the strain was cultured at 52°C. This was inoculated into three 1-L medium bottles each containing 50 mL of liquid A medium and cultured to obtain a total of 150 mL of seed culture. This seed culture was added to liquid A medium in a 3-L jar fermenter to make a total medium volume of 1.5 L. After 8 hours of culture, about 25 mL of the bacterial cell culture liquid was collected.

The collected culture liquid was centrifuged, and the supernatant was passed through a filter with a pore size of 0.22 µm to completely remove bacterial cells. The filtered supernatant was concentrated by centrifugation using an ultrafiltration unit (Amicon Ultra 15 mL, 10,000 MWCO) until the volume was reduced to about one-eightieth of its initial volume.

A volume of 4 µL of this concentrated culture supernatant was sampled and analyzed by 5-20% SDS-PAGE, and many proteins were detected by Coomassie Brilliant Blue G-250 staining. Using molecular weight markers as a guide, a gel portion corresponding to a molecular weight of about 15 to 40 kDa and a gel portion corresponding to a molecular weight of about 40 to 75 kDa were cut out, and each sliced into about 1 mm square pieces. The former was called Sample 1, and the latter was called Sample 2.

### (1-2) Protein identification by liquid chromatography-mass spectrometry

The identification of proteins contained in Samples 1 and 2 was outsourced to MS Bioworks, Inc. (Ann Arbor, Michigan, U.S.A.) and performed according to the following procedure.

The polyacrylamide gel portions, which are protein samples, were washed with 25 mM ammonium carbonate solution and then dehydrated by immersion in acetonitrile. This was followed by reduction treatment with 10 mM dithiothreitol and alkylation (carbamidomethylation) with 50 mM iodoacetamide, which cleaved the disulfide bonds within the proteins and prevented these bonds from forming again. Each gel portion was digested with porcine trypsin (manufactured by Promega) at 37°C for 4 hours, and the reaction was stopped with formic acid. Trypsin-digested peptide mixtures eluted from the gel portions were collected and used for analysis.

Each trypsin-digested peptide mixture was analyzed using nanoLC-MS/MS system composed of ACQUITY UPLC M-Class liquid chromatography system (manufactured by Waters) combined with Orbitrap Fusion Lumos mass spectrometer (manufactured by Thermo Fisher Scientific). The liquid chromatography analysis was performed using an analytical column with a column internal diameter of 75 µm packed with XSelect CSH C18 resin (2.4 µm in diameter) at a flow rate of 350 nL per minute. The mass spectrometer was operated at a resolution of 60,000 FWHM for MS analysis and 15,000 FWHM for MS/MS analysis. MS and MS/MS modes were alternately switched every 3 seconds while data were acquired.

MS and MS/MS data obtained from the mass spectrometer were processed using Mascot (software manufactured by Matrix Science). That is, using Mascot, MS/MS spectra including measured masses of multiple fragment ions generated by cleaving and fragmenting each trypsin-digested peptide were compared with and matched against hypothetical MS/MS spectra calculated from the amino acid sequences of the proteins encoded in the genome of *Hydrogenophilus thermoluteolus,* which are available from the protein database (GenBank). According to this procedure (known as MS/MS ion search), proteins to which the amino acid sequences of the trypsin-digested peptides were assigned were identified.

The settings used for comparison and matching using Mascot is as follows.
Cleavage enzyme: Trypsin
Organism species in the database: *Hydrogenophilus thermoluteolus*
Fixed modifications of amino acids: Carbamidomethylation (cysteine)
Variable modifications of amino acids: Oxidation (methionine), acetylation (N-terminus), pyroglutamation (N-terminal glutamine), deamination (aspartic acid, glutamine)
Trypsin-digested peptide mass tolerance: 10 ppm or less MS/MS fragment ion mass tolerance: 0.02 Da or less

The analysis was performed under the above conditions, and a protein to which amino acid sequences obtained from at least two independent MS/MS spectra were assigned was identified as a protein present in the culture supernatant when the false discovery rate (FDR) was 1% or less as calculated on the basis of mass measurement errors. The results of the analysis showed that 337 proteins were identified from Sample 1 including proteins of about 40 kDa or less and 206 proteins were identified from Sample 2 including proteins of about 40 kDa or more. The total number of identified proteins, excluding duplicates, was 502.

### (1-3) Identification of secretory proteins

The culture supernatant is expected to contain not only proteins secreted by bacterial cells, but also large amounts of proteins from disrupted bacterial cells such as those lysed during culture. That is, all of the proteins of about 15 kDa or more that were contained in the culture supernatant and identified by Mascot are not secretory proteins.

Therefore, the 502 identified proteins in the culture supernatant were searched for the presence of secretory signal peptides using their amino acid sequences encoded in the genome of *Hydrogenophilus thermoluteolus* with the secretory signal peptide prediction programs PrediSi (http://www.predisi.de) and SignalP (https://services.healthtech.dtu.dk/services/SignalP-6.0/). This search revealed that 79 of the 502 proteins were predicted to contain secretory signal peptides.

PrediSi is described in "Hiller et al., Nucleic Acids Research, 32, W375-W379 (2004)", and SignalP is described in "Teufel et al., Nature Biotechnology, 40, 1023-1025 (2022)". The number of citations of these documents are 193 and 201, respectively, indicating that these programs are highly reliable.

For more accuracy, the present inventor compared and scrutinized the amino acid sequences of the secretory proteins predicted by the secretory signal peptide prediction programs and the amino acid sequences of peptides that were present in the culture supernatant and identified by mass spectrometry. Secretory signal peptides are usually removed at the time of secretion and are generally not present in the proteins identified in the culture supernatant by mass spectrometry. Therefore, even a protein predicted to be a secretory protein by the secretory signal peptide prediction programs is not considered as a secretory protein when any peptide present in the culture supernatant is assigned to the N-terminal region of the predicted secretory protein, that is, when the N-terminal region of the amino acid sequence of the predicted secretory protein contains an amino acid sequence matching that of any peptide present in the culture supernatant.

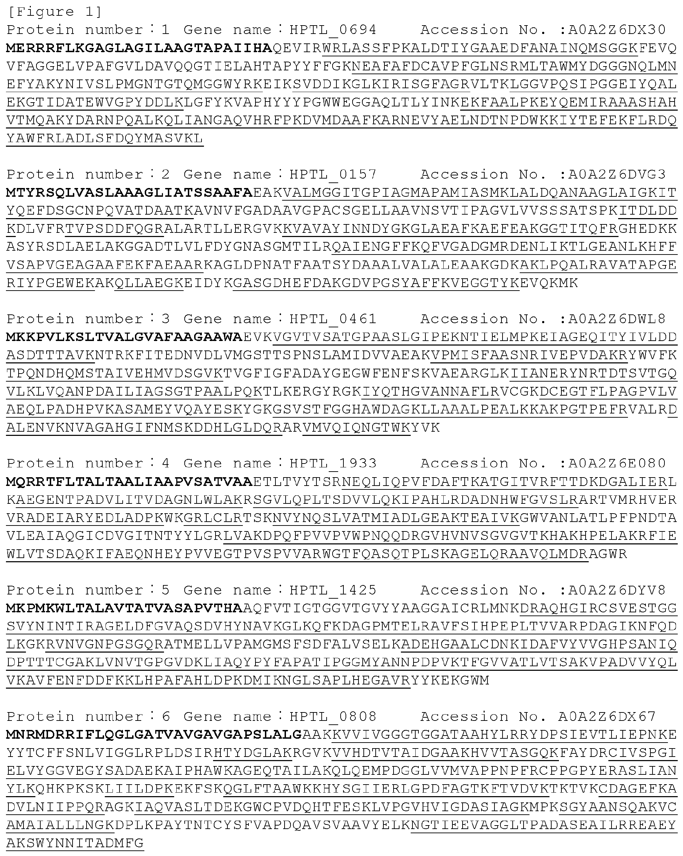

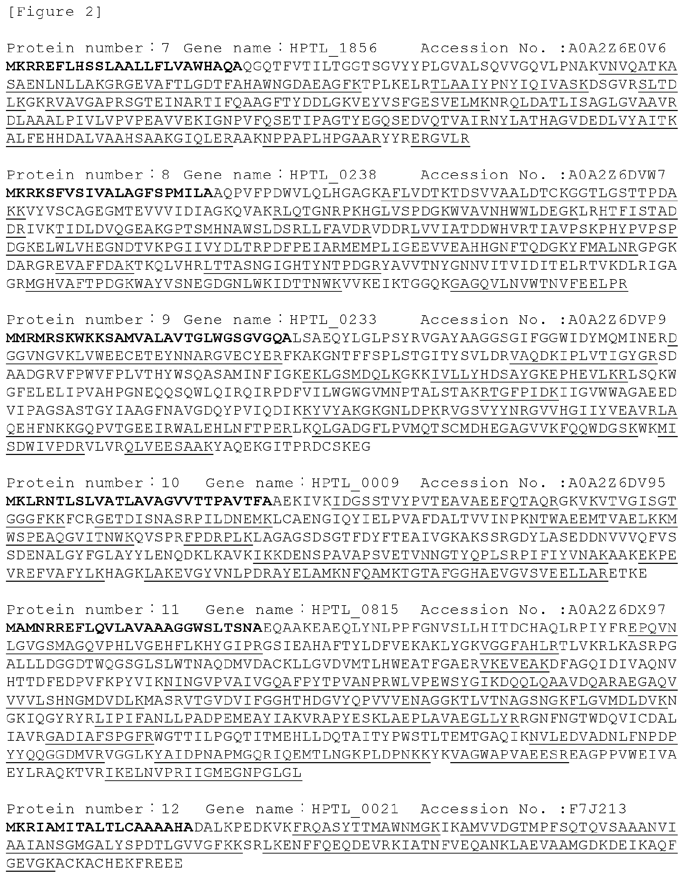

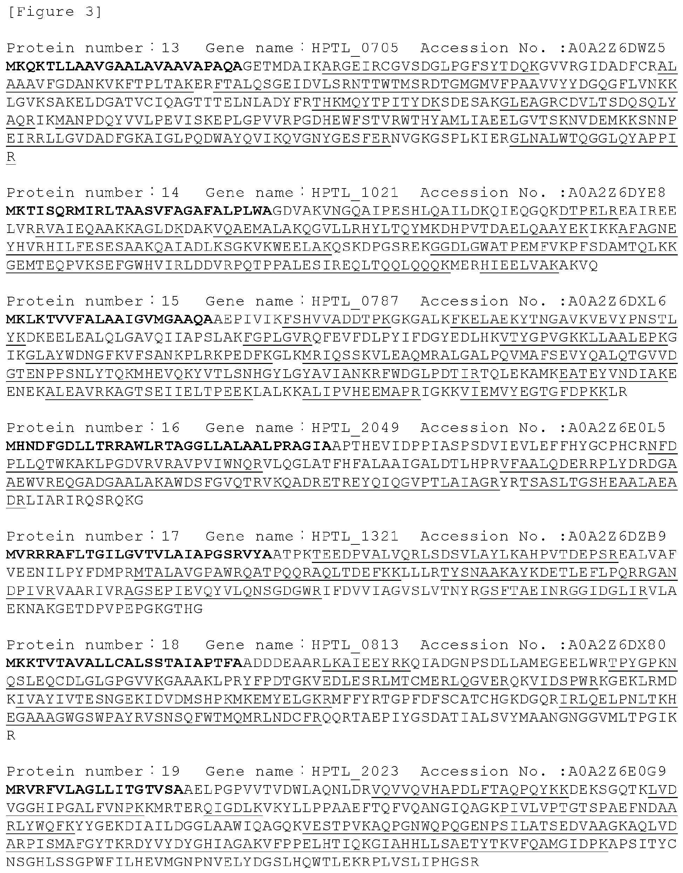

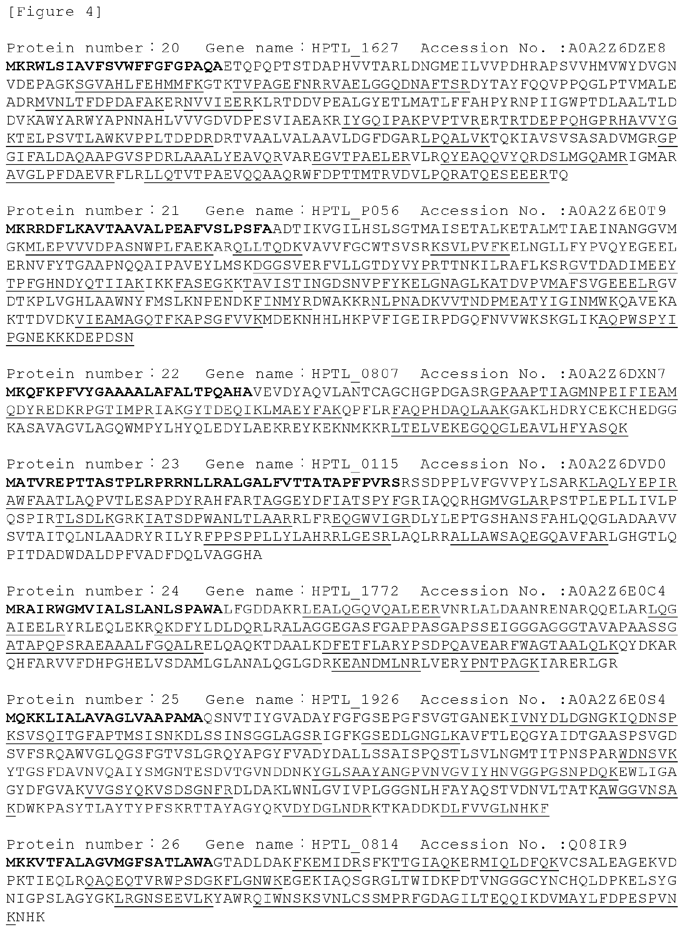

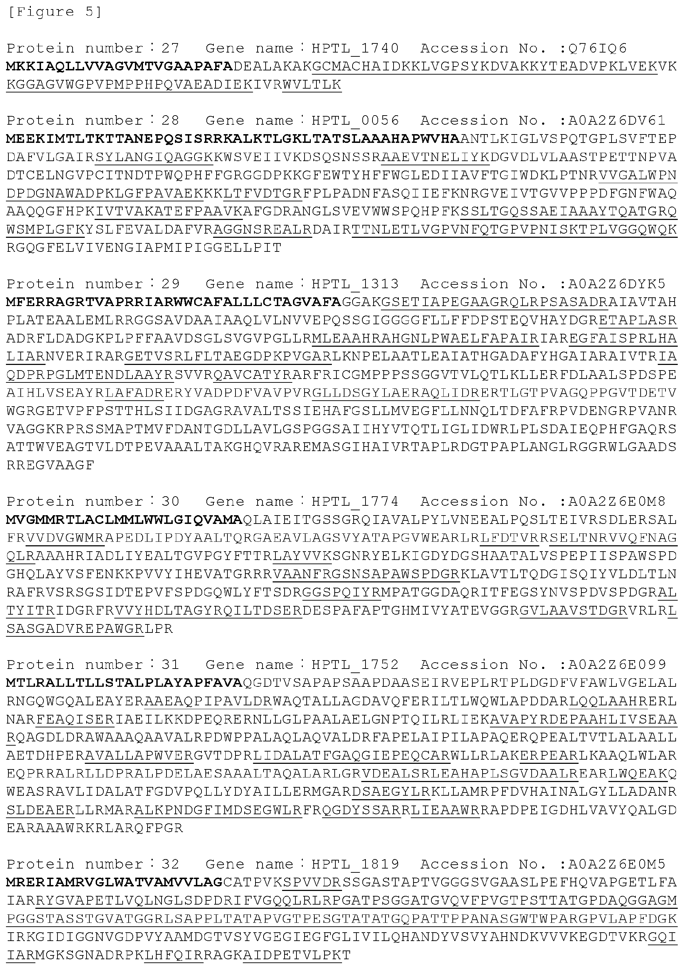

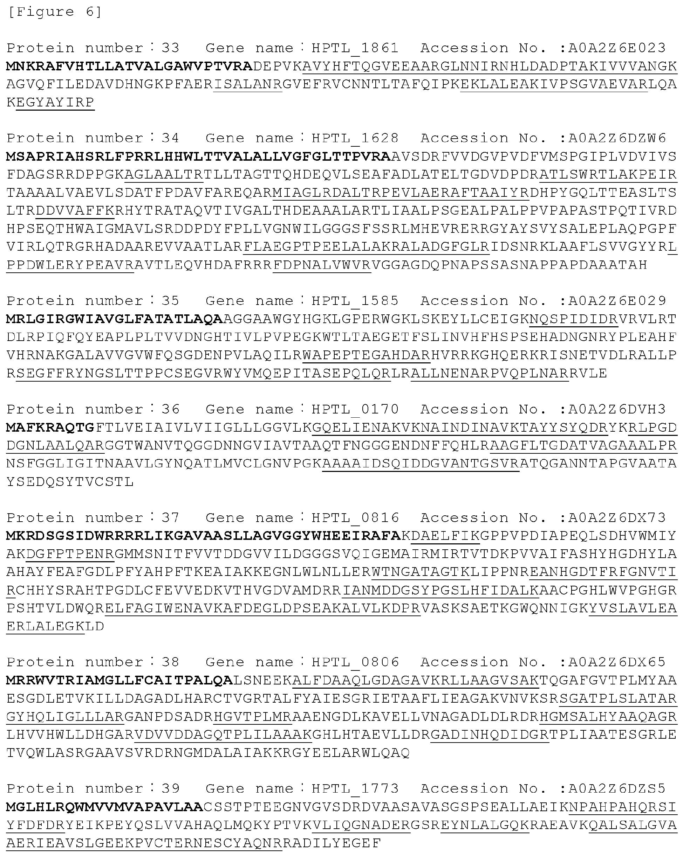

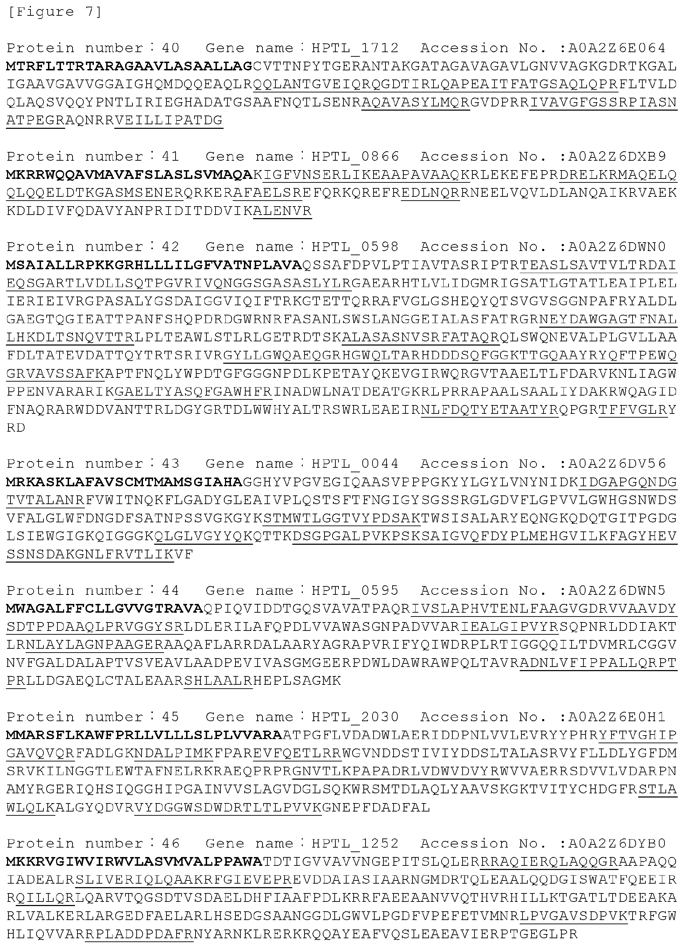

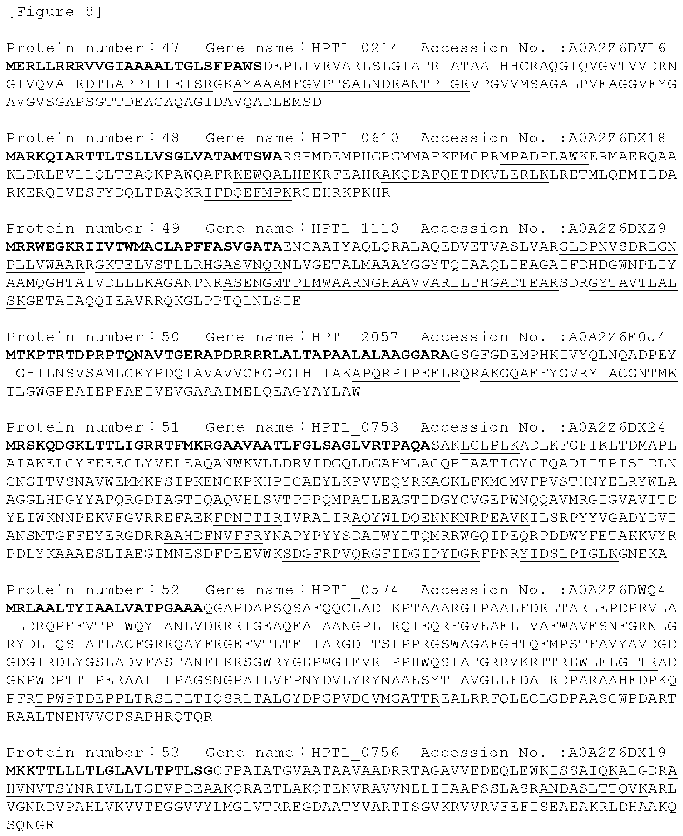

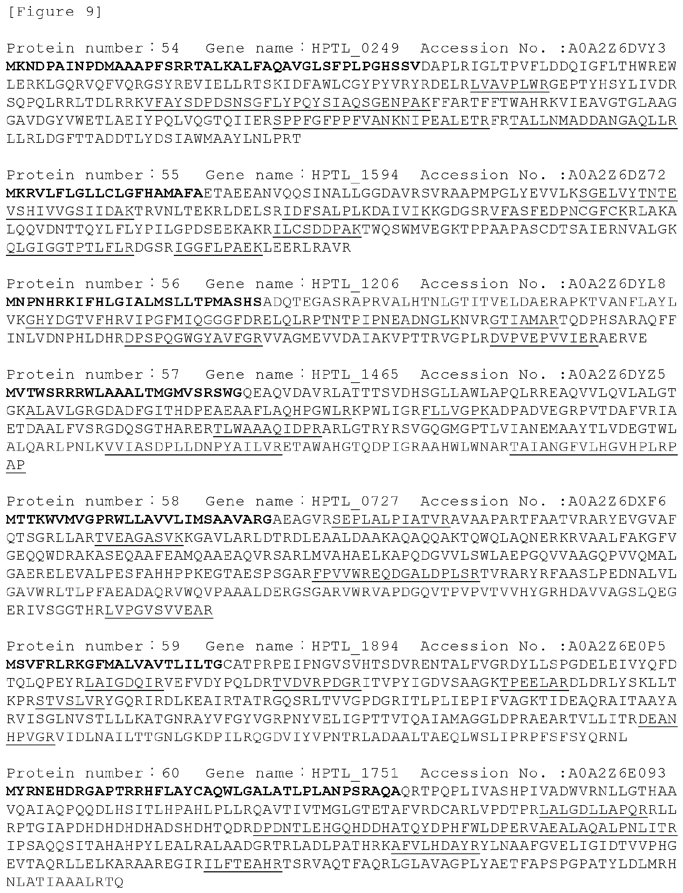

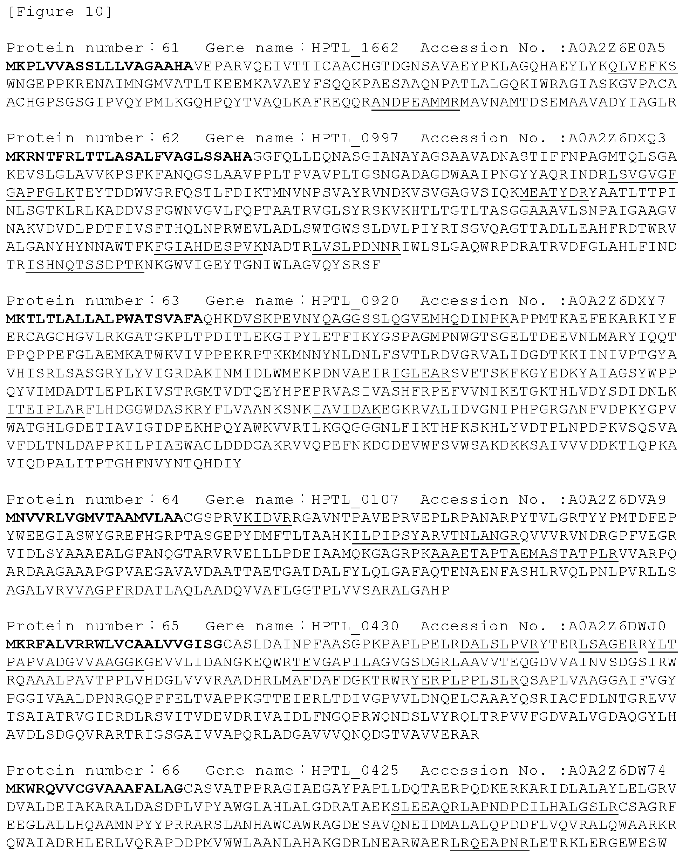

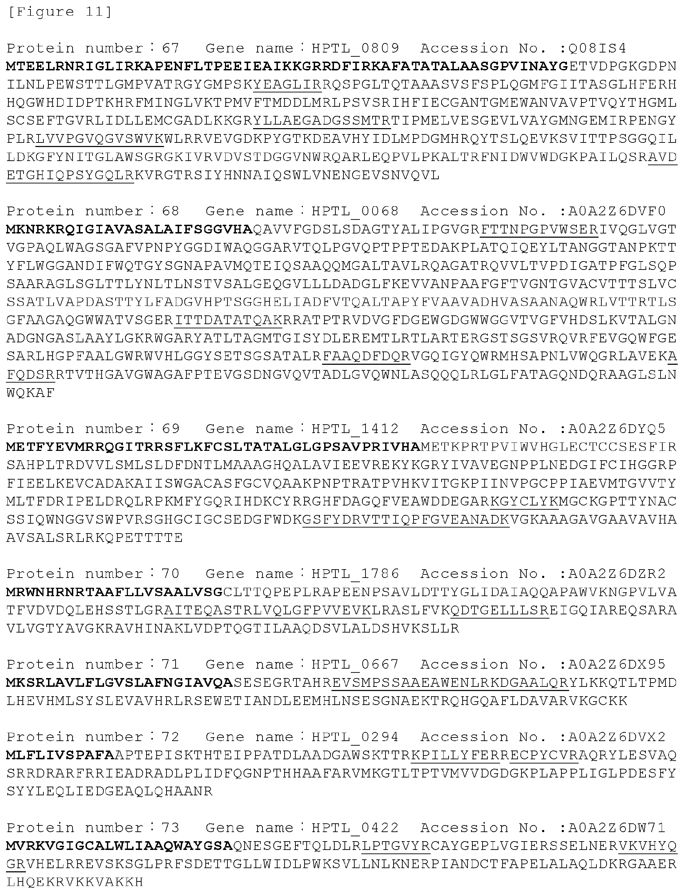

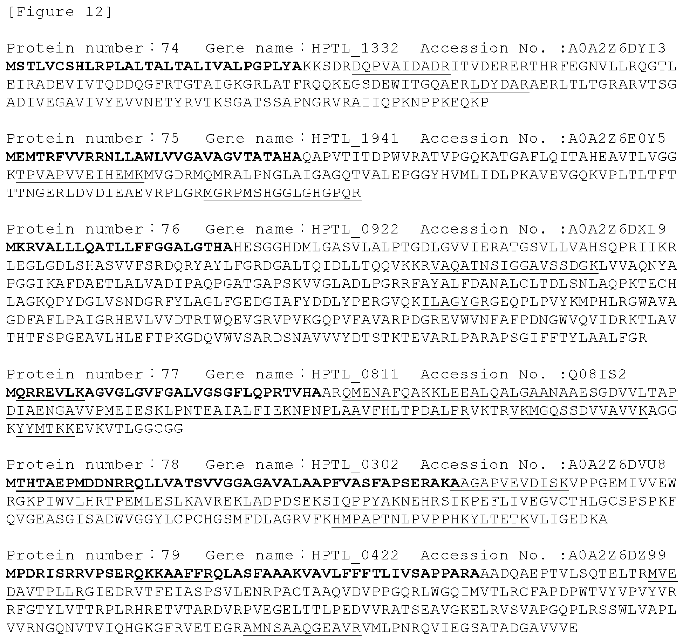

The matching results are shown in Figs. 1 to 12. The amino acid sequences of the predicted secretory signal sequences are marked in bold, and the amino acid sequences that were present in the culture supernatant samples and identified by mass spectrometry are marked with underlines. The N-terminal regions of the amino acid sequences of proteins Nos. 1 to 76 have no amino acid sequences matching those of peptides present in the culture supernatant. Therefore, these proteins were identified as secretory proteins. The amino acid sequences marked in bold in the N-terminal regions of proteins Nos. 1 to 76 are the amino acid sequences of the secretory signal peptides Nos. 1 to 76 predicted by the secretory signal peptide prediction programs, respectively.

The amino acid sequences of the secretory signal peptides Nos. 1 to 76 contained in the secretory proteins Nos. 1 to 76 (the odd-numbered sequences of SEQ ID NOs: 1 to 152) and one exemplary set of the nucleotide sequences encoding these amino acid sequences (the even-numbered sequences of SEQ ID NOs: 1 to 152) are shown in Tables 1 to 4.

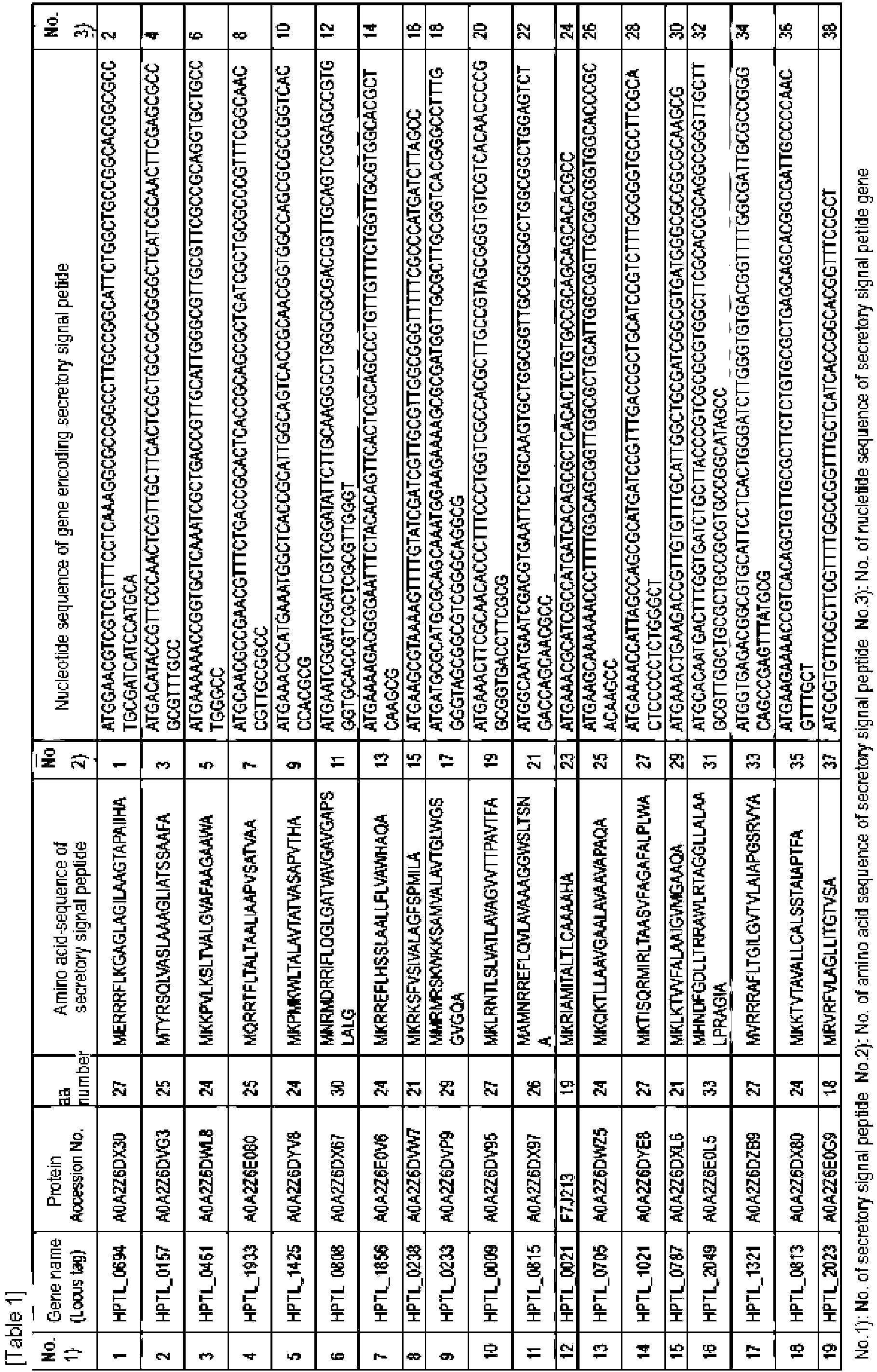

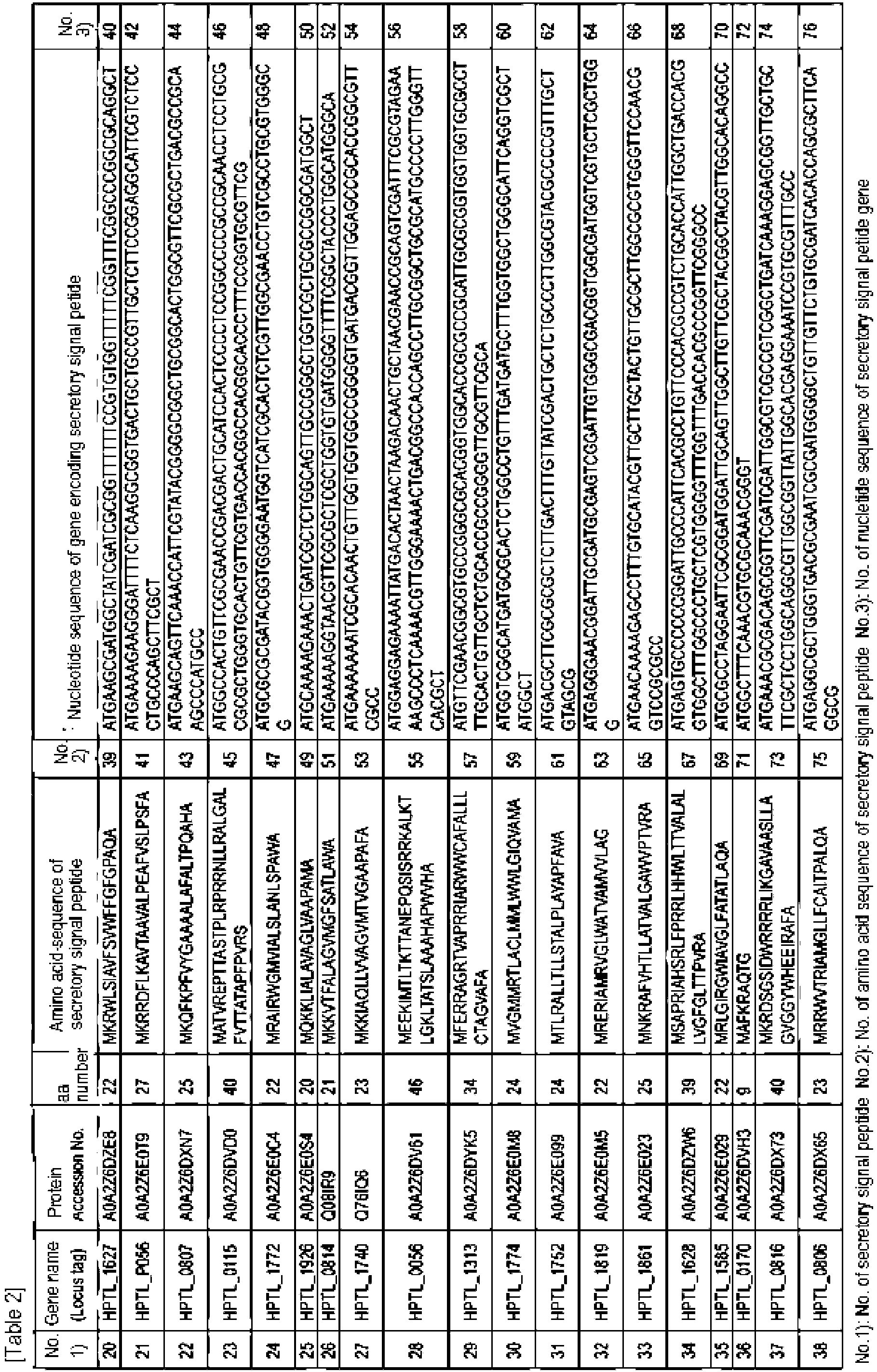

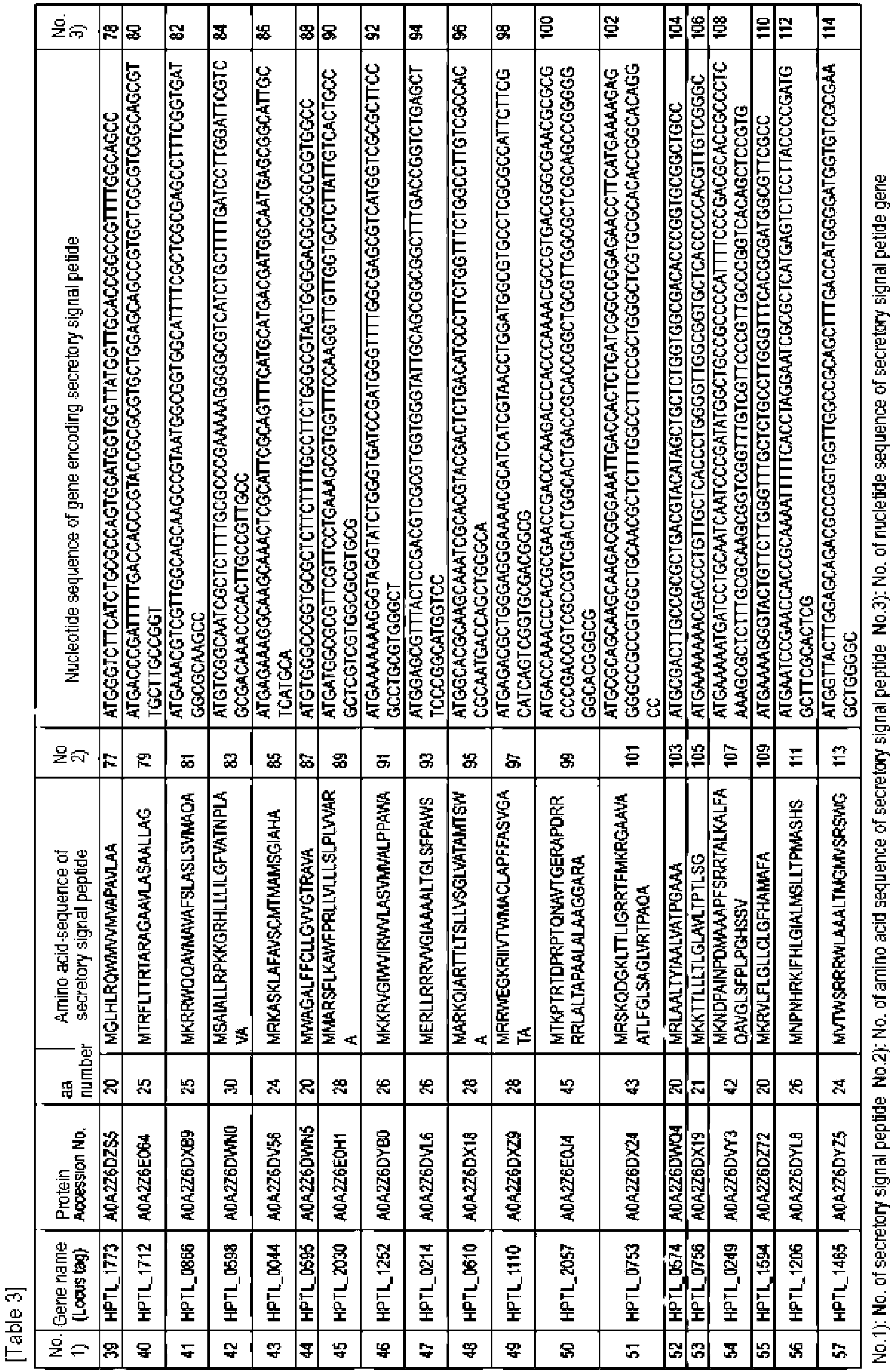

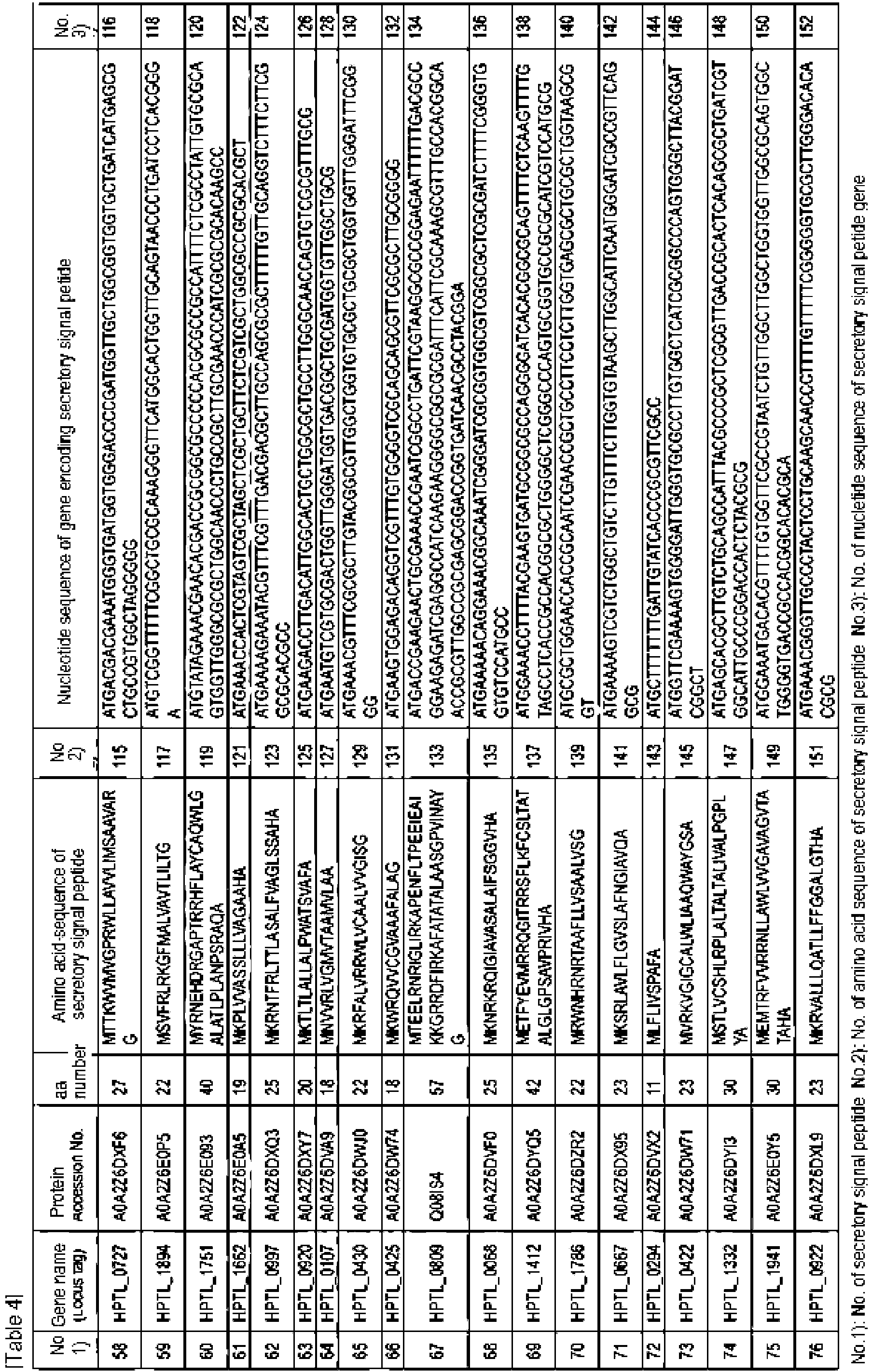

On the other hand, the N-terminal region of each of three proteins Nos. 77 to 79 has an amino acid sequence matching that of a peptide present in the culture supernatant. Therefore, these were considered unlikely to be secretory proteins. This also indicates that there is no particular difficulty in assigning the amino acid sequence of a peptide present in the culture supernatant to the amino acid sequence near the N-terminus of a protein.

When two or more of the amino acid sequences of peptides present in the sample are assigned with high mass accuracy to the amino acid sequence of a protein, this protein can be generally determined to be present in the sample. For example, proteins Nos. 66 and 69 to 76 each have two amino acid sequences matching those of peptides present in the culture supernatant (two consecutive underlines are drawn in protein No. 71). Additionally, considering that the false discovery rate (FDR) calculated on the basis of mass measurement errors is 1% or less, these proteins can be considered to be present in the culture supernatant.

The secretory proteins Nos. 1 to 76 shown in Figs. 1 to 12 were identified from two or more MS/MS spectra. Particularly, 10 or more spectra were assigned to the secretory proteins Nos. 1 to 46 having the respective signal peptides consisting of the amino acid sequences of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, and 91. More particularly, 40 or more spectra were assigned to the secretory proteins Nos. 1 to 16 having the respective signal peptides consisting of the amino acid sequences of SEQ ID NOs: 1, 3, 5, 7, 9, 11, 13, 15, 17, 19, 21, 23, 25, 27, 29, and 31. The number (spectral count) of MS/MS spectra assigned to an identified protein is known to have a strong positive correlation with the amount of the protein, and this correlation is widely used in relative quantification methods.

From this, it can be concluded that among the 76 signal peptides shown in Tables 1 to 4, the signal peptides Nos. 1 to 46 consisting of the amino acid sequences of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, and 91 are derived from proteins secreted in greater amounts as compared with other signal peptides and are more preferable signal peptides. It can also be concluded that the signal peptides Nos. 1 to 16 consisting of the amino acid sequences of SEQ ID NOs: 1, 3, 5, 7, 9, 11, 13, 15, 17, 19, 21, 23, 25, 27, 29, and 31 are derived from proteins secreted in even greater amounts and are even more preferable signal peptides.

### (2) Construction of plasmid vector (pCAMO-6)

The method for constructing plasmid vector pCAMO-6 used for the introduction of a gene encoding a fusion protein of a secretory signal sequence and a target protein to be expressed is described below.

### (2-1) Preparation of tac promoter-containing DNA fragment

Plasmid pMAL-c5X manufactured by New England Biolabs was used as a template to amplify a DNA fragment containing tac promoter by PCR using a pair of primers shown below. Hereinafter, unless otherwise stated, PCR was performed in the usual manner using 2720 Thermal Cycler manufactured by Thermo Fisher Scientific Inc. and using KOD FX Neo (manufactured by Toyobo Co., Ltd.) as a reaction reagent.

### Primers for amplification of tac promoter

(a-1) 5 '-TTTTATAACCCGGGCCATCGACTGCACGGTGCACC-3' (SEQ ID NO: 153)
(b-1) 5'-TGCTAGCACTGTTTCCTGTGTGAAATTGTTATCCG-3' (SEQ ID NO: 154)

The primer (a-1) contains a SmaI restriction enzyme site as marked with an underline.

The resulting reaction mixture was subjected to agarose gel electrophoresis. The results showed that an about 0.3-kbp DNA fragment corresponding to tac promoter was detected. A gel portion containing this DNA fragment was cut out of the agarose gel, and the gel portion was freeze-thawed to recover the DNA fragment.

### (2-2) Preparation of multicloning site-containing DNA fragment

For the preparation of a DNA fragment containing a multicloning site, the former and latter halves of the multicloning site were separately produced by PCR using the respective pairs of primers shown below. This method does not use any template DNA and allows each pair of primers to anneal together in their 3' terminal regions and elongate to form a double-stranded DNA.

### Primers for preparation of former half of multicloning site

(a-2) 5'-GGAAACAGTGCTAGCAGATCTGGAGGAGAAACGCATAT-3' (SEQ ID NO: 155)

(b-2) 5'-
CAGTGCGGCCGCAAGCTTGTCGACGGAGCTCGAATTCGGATCCGATATCAGCATATGCGTTTC
TCCTCCAGA-3' (SEQ ID NO: 156)

The 3' terminal 20-nucleotide sequences of the primers (a-2) and (b-2) are complementary to each other.

### Primers for preparation of latter half of multicloning site

(a-3) 5'-
ACAAGCTTGCGGCCGCACTGCAGCACCATCACCACCATCATTGATAAGATCCGGCTGCTAACA
AAGCCCGAAAGGAAGCT-3' (SEQ ID NO: 157)

(b-3) 5'-
TATTTGAATCGAGTTATTGCTCAGCGGTGGCAGCAGCCAACTCAGCTTCCTTTCGGGCTTTGT
-3' (SEQ ID NO: 158)

The 3' terminal 20-nucleotide sequences of the primers (a-3) and (b-3) are complementary to each other.

The resulting reaction mixture was subjected to agarose gel electrophoresis. The results showed that about 0.1-kbp DNA fragments corresponding to the former and latter halves of the multicloning site were detected. Gel portions containing the DNA fragments were cut out of the agarose gel, and the gel portions were freeze-thawed to recover the DNA fragments.

Overlap extension PCR was performed through use of the recovered DNA fragments corresponding to the former and latter halves of the multicloning site as templates. The 5' terminal 20-nucleotide sequences of the primers (b-2) and (a-3) used to amplify these template DNA fragments are complementary to each other. For the overlap extension PCR, a combination of the primers (a-2) and (b-3) shown above was used to construct a DNA containing the multicloning site.

The resulting reaction mixture was subjected to agarose gel electrophoresis. The results showed that an about 0.2-kbp DNA fragment corresponding to the multicloning site was detected. This was recovered from the agarose gel.

### (2-3) Preparation of DNA fragment containing rrnB terminator joined to origin of replication of pUC19

Plasmid pMAL-c5X manufactured by New England Biolabs was used as a template to amplify a DNA fragment containing rrnB terminator by PCR using a pair of primers shown below.

### Primers for amplification of rrnB terminator

(a-4) 5'-TAACTCGATTCAAATAAAACGAAAGGCTCAGTCGA-3' (SEQ ID NO: 159)
(b-4) 5'-CCTAGATCCGCGGAGTTTGTAGAAACGCAAAAAGG-3' (SEQ ID NO: 160)

In addition, plasmid pUC19 was used as a template to amplify a DNA fragment containing an origin of DNA replication by PCR using a pair of primers shown below.

### Primers for amplification of origin of DNA replication of pUC19

(a-5) 5'-ACAAACTCCGCGGATCTAGGTGAAGATCCTTTTTG-3' (SEQ ID NO: 161)
(b-5) 5'-CGTCCGCGGCCAGCAAAAGGCCAGGAACCGTAAAA-3' (SEQ ID NO: 162)

The resulting reaction mixtures were each subjected to agarose gel electrophoresis. The results showed that an about 0.3-kbp DNA fragment corresponding to rrnB terminator and an about 0.8-kbp DNA fragment corresponding to the origin of DNA replication of pUC19 were detected. Gel portions containing the DNA fragments were cut out of the agarose gel, and the gel portions were freeze-thawed to recover the DNA fragments.

Overlap extension PCR was performed through use of the recovered DNA fragments corresponding to rrnB terminator and the origin of DNA replication of pUC19 as templates. The 5' terminal 20-nucleotide sequences of the primers (b-4) and (a-5) used to amplify these template DNA fragments are complementary to each other. For the overlap extension PCR, a combination of the primers (a-4) and (b-5) was used to construct a DNA fragment containing rrnB terminator joined to the origin of replication of pUC19.

The resulting reaction mixture was subjected to agarose gel electrophoresis. The results showed that an about 1.0-kbp DNA fragment corresponding to a DNA containing rrnB terminator joined to the origin of replication of pUC19 was detected. This was recovered from the agarose gel.

### (2-4) Preparation of DNA fragment containing neomycin/kanamycin resistance gene

Plasmid pK18mobsacB (GenBank: FJ437239.1) (Gene, 145, 69-73 (1994)), which contains a neomycin/kanamycin resistance gene (hereinafter sometimes referred to as "nptII") sequence, was used as a template to amplify a DNA fragment containing the nptII gene sequence by PCR using a pair of primers shown below.

### Primers for amplification of nptII gene

(a-6) 5'-TTGCTGGCCGCGGACGTAGAAAGCCTGTCCGCAGA-3' (SEQ ID NO: 163)
(b-6) 5 '-GGCCCGGGTTATAAAAGCCAGTCATTAGGCCTATC-3' (SEQ ID NO: 164)

The primer (b-6) contains a SmaI restriction enzyme site as marked with an underline.

The resulting reaction mixture was subjected to agarose gel electrophoresis. The results showed that an about 1.0-kbp DNA fragment corresponding to the nptII gene was detected. This was recovered from the agarose gel.

### (2-5) Construction of circular plasmid

For the DNA fragments prepared in the above (1-1) to (1-4), the terminal region of the DNA fragment in (1-1) has a 16-bp sequence homologous to the terminal regions of the DNA fragments in (1-4) and (1-2); the terminal region of the DNA fragment in (1-2) has a 16-bp sequence homologous to the terminal regions of the DNA fragments in (1-1) and (1-3); the terminal region of the DNA fragment in (1-3) has a 16-bp sequence homologous to the terminal regions of the DNA fragments in (1-2) and (1-4); and the terminal region of the DNA fragment in (1-4) has a 16-bp sequence homologous to the terminal regions of the DNA fragments in (1-3) and (1-1).

Thus, the DNA fragments in (1-1), (1-2), (1-3), and (1-4) can be joined together into the form of a circular DNA by Gibson Assembly (Gibson et al., Nature Methods 6(5): 343-345), a DNA ligation method that does not require any restriction enzyme or ligase. Gibson Assembly was performed using a Gibson Assembly Master Mix manufactured by New England Biolabs to join the DNA fragments prepared in (1-1) to (1-4) into a circular DNA. *Escherichia coli* JM109 was transformed with the resulting reaction mixture by calcium chloride method, and applied to solid LB medium containing 50 µg/mL kanamycin. Strains grown on the medium were cultured in liquid medium in the usual manner. Plasmid DNA was extracted from the culture liquid and reacted with the restriction enzyme SmaI. This reaction mixture was subjected to agarose gel electrophoresis. The results showed that an about 2.3-kbp DNA fragment was observed, which corresponds in size to a single piece of DNA formed by joining the DNA fragments prepared in (1-1) to (1-4), demonstrating successful assembly of the DNA fragments.

This circular plasmid DNA, which is replicable in *Escherichia coli,* was named pCAMO-1.

### (2-6) Construction of plasmid vector (pCAMO-4)

*Hydrogenophilus thermoluteolus* strain TH-1 was inoculated with a platinum loop into 5 mL of liquid A medium in a test tube. The test tube was filled with a gas mixture of H₂:O₂:CO₂ = 7.5:1:1.5, and the strain was cultured at 50°C.

Endogenous plasmid pTH1 (Microbiol Resour Announc. 2018 Aug 16; 7(6)) was prepared from the culture liquid by the conventional alkaline-SDS method.

The prepared pTH-1 of about 66 kbp in size was cut with restriction enzymes ScaI and PvuII, and the plasmid pCAMO-1 was cut with restriction enzyme SmaI. Both of them were ligated together by T4 DNA ligase (manufactured by Takara Bio Inc.).

*Hydrogenophilus thermoluteolus* strain TH-1 (NBRC 14978) was transformed with the resulting ligation mixture by electric pulse method (electroporation), applied to solid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, 4.0 mg of CoCl₂·6H₂O, and 15 g of agar were dissolved in 1 L of distilled water (pH 7.0)] containing 50 µg/mL kanamycin, and cultured in a chamber filled with a gas mixture of H₂:O₂:CO₂ = 7.5:1:1.5 at 50°C for 60 hours.

Seven strains grown on the solid A medium were separately inoculated with a platinum loop into 5 mL of liquid A medium containing 50 µg/mL kanamycin per test tube. The test tubes were filled with a gas mixture of H₂:O₂:CO₂ = 7.5:1:1.5, and the strains were cultured with shaking at 52°C. Plasmid DNA was extracted from the culture liquid and subjected to agarose gel electrophoresis. The results showed that all of the 7 strains had the same plasmid DNA of about 5.5 kbp in size.

The extracted plasmid was used as a template to amplify a DNA fragment containing the endogenous plasmid pTH1 inserted in the SmaI restriction enzyme site of pCAMO-1 by PCR using a pair of primers shown below. The pair of primers correspond to the regions at both sides of the SmaI restriction enzyme site of pCAMO-1.

### Primers for amplification of inserted pTH1 DNA fragment

(a-7) 5'-AATTGTCAGATAGGCCTAATGACTGGCTTTTATAA-3' (SEQ ID NO: 165)
(b-7) 5'-TGACGCCAGAAGCATTGGTGCACCGTGCAGTCGAT-3' (SEQ ID NO: 166)

The resulting reaction mixture was subjected to agarose gel electrophoresis. The results showed that an about 3.2-kbp DNA fragment was detected. That is, the obtained plasmid contains an about 3.2-kbp DNA fragment corresponding to a part of pTH1, which has been inserted into the SmaI restriction enzyme site of the pCAMO-1 plasmid. Since this about 3.2-kbp DNA fragment contains an origin of replication that functions in *Hydrogenophilus thermoluteolus* cells, the obtained plasmid is replicable in *Hydrogenophilus thermoluteolus* cells.

The constructed plasmid was named pCAMO-4.

### (2-7) Construction of plasmid vector (pCAMO-5)

Equal moles of a pair of oligonucleotides shown below were mixed, and the mixture was gently cooled from 98°C to 20°C, yielding a double-stranded DNA in which the oligonucleotides were annealed. Both ends of this DNA fragment are equivalent to the protruding ends generated by cleavage with restriction enzymes BglII and NdeI. This DNA fragment and a digested DNA fragment prepared by cutting pCAMO-4 with restriction enzymes BglII and NdeI were ligated together by T4 DNA ligase.
(a-8) 5'-GATCTGGAGGAGAAACGCA-3' (SEQ ID NO: 167)
(b-8) 5'-TATGCGTTTCTCCTCCA-3' (SEQ ID NO: 168)

The constructed plasmid was named pCAMO-5.

### (2-8) Construction of plasmid vector (pCAMO-6)

The endogenous plasmid pTH-1 was used as a template to amplify a DNA fragment containing mazF gene encoding a plasmid stabilization factor by PCR using a pair of primers shown below.

### Primers for amplification of mazF gene

(a-9) 5'-GAGGCCATCTAGGCCATGAGTAAGTCTGACGGAAC-3' (SEQ ID NO: 169)
(b-9) 5 '-ATCCGGCACCCATATCTGAACCGGACGCAAACCCG-3' (SEQ ID NO: 170)

The endogenous plasmid pTH-1 was used as a template to amplify a DNA fragment containing mazE gene encoding a plasmid stabilization factor by PCR using a pair of primers shown below.

### Primers for amplification of mazE gene

(a-10) 5'-TTCAGATATGGGTGCCGGATACCCGCCGCCCGGGC-3' (SEQ ID NO: 171)
(b-10) 5'-AAGGCCTTCATGGCCTTATTTCGCGATTCCCAAGA-3' (SEQ ID NO: 172)

The 3' terminal region of the mazF-containing DNA fragment has a 20-bp sequence homologous to the 5' terminal region of the mazE-containing DNA fragment. Thus, the mazF-containing DNA fragment and the mazE-containing DNA fragment can be joined by PCR. The DNA fragments prepared above were mixed, and for the amplification of the joined DNA fragments, PCR was performed using the primers (a-9) and (b-10).

The resulting reaction mixture was subjected to agarose gel electrophoresis. The results showed that an about 0.7-kbp DNA fragment corresponding to a DNA formed by joining the mazF-containing DNA fragment and the mazE-containing DNA fragment was detected. This was recovered from the agarose gel.

The prepared DNA fragment of about 0.7 kbp in size and the plasmid pCAMO-5 were each cut with a restriction enzyme, SfiI. Both of them were ligated together by T4 DNA ligase.

The constructed plasmid was named pCAMO-6.

### (3) Construction of gene expression plasmids for protein secretion

### (3-1) Preparation of secretory signal peptide gene fragments

To construct vectors for expressing fusion genes in which the genes consisting of the odd-numbered nucleotide sequences of SEQ ID NOs: 1 to 152 are separately joined to the gene of a target protein to be secreted, DNA fragments containing the odd-numbered nucleotide sequences of SEQ ID NOs: 1 to 152 are prepared as follows.

Oligonucleotides corresponding to sense strands are prepared by adding GCAT to the 5' end of each of the odd-numbered nucleotide sequences of SEQ ID NOs: 1 to 152, and oligonucleotides corresponding to antisense strands are prepared by adding CGAC to the 5' end of each of the complementary nucleotide sequences of the odd-numbered nucleotide sequences of SEQ ID NOs: 1 to 152. For illustrative purposes, SEQ ID NO: 1 is used as an example, and the prepared oligonucleotide pair is shown below.

Two oligonucleotides corresponding to a pair of sense and antisense strands are mixed, phosphorylated at their 5' ends using T4 polynucleotide kinase, kept at 95°C for 10 minutes, and slowly cooled so that they are annealed to form a double strand. This reaction can also be performed by mixing a number of oligonucleotides, for example, 152 oligonucleotides for producing 76 DNA fragments containing the odd-numbered nucleotide sequences of SEQ ID NOs: 1 to 152, respectively. In this case, a library of secretory signal peptide genes can be created. The resulting double-stranded gene fragments are separately inserted into expression vector pCAMO-6.

A gene expression plasmid is constructed using Golden Gate system (NEB).

Here, a green fluorescent protein (muGFP) is used as the target protein to be secreted.

Plasmid vector pCAMO-6 is amplified by PCR for subsequent DNA ligation using Golden Gate system. The primers shown below are used for PCR.

### Primers for amplification of plasmid vector pCAMO-6

(a-11) 5'-GGCTACGGTCTCAATTCGAGCTCCGTCGACAAGC-3' (SEQ ID NO: 175)
(b-11) 5'-GGCTACGGTCTCGCATATGCGTTTCTCCTCCAGATC-3' (SEQ ID NO: 176)

A reaction mixture is incubated with a restriction enzyme, DpnI, at 37°C for 30 minutes to digest the vector DNA used as a template, followed by amplification. After agarose gel electrophoresis, a gel portion containing the amplified linear vector DNA fragment is cut out, and the vector DNA fragment is recovered from the gel portion with a GEL/PCR Purification Mini Kit (FAVORGEN).

A DNA (SEQ ID NO: 179) in which a DNA encoding the green fluorescent protein muGFP is joined to terminal sequences required for cloning (GCGGGTCTCAT (SEQ ID NO: 177) before the start codon, GAATTCGGAGACCCGC (SEQ ID NO: 178) after the stop codon) is obtained by gene synthesis (outsourced to Twist Biotechnology).

The synthesized DNA fragment containing muGFP gene and the linear pCAMO-6 DNA fragment are ligated together using an NEBridge Golden Gate Assembly Kit (BsaI-HF v2) manufactured by New England Biolabs. At this time, the restriction enzyme BsaI included in the kit cleaves the sites near both ends of each DNA fragment to generate sticky ends (TATG, GAAT), which are then used for ligation by T4 DNA ligase to join the DNAs.

*Escherichia coli* strain JM109 is transformed with the resulting reaction mixture by heat shock method, applied to LB medium containing 50 µg/mL kanamycin, and cultured at 37°C for 24 hours.

Strains grown on the LB medium are separately inoculated with a platinum loop into 5 mL of liquid LB medium containing 50 µg/mL kanamycin per test tube, and cultured with shaking at 37°C. Plasmid DNA is extracted from the culture liquid. The analysis of the sequence of the gene inserted in the plasmid is outsourced to Eurofins Genomics K.K. and performed by Sanger method to confirm that the sequence of the gene is identical to the corresponding sequence in the database. This plasmid is named pCAMO-6-muGFP.

The plasmid vector pCAMO-6-muGFP is amplified by PCR for subsequent ligation of a secretory signal gene fragment using Golden Gate system. The primers shown below are used for PCR.

### Primers for amplification of plasmid vector pCAMO-6-muGFP

(a-12) 5'-GGCTACGGTCTCAGTCGAAAGGCGAAGAACTCTTC-3' (SEQ ID NO: 180)
(b-12) 5'-GGCTACGGTCTCGATGCGTTTCTCCTCCAGATC-3' (SEQ ID NO: 181)

The BsaI restriction enzyme recognition sequences (GGTCTC) are marked with underlines, and the sticky ends generated by BsaI cleavage are marked with double underlines.

A reaction mixture is incubated with the restriction enzyme DpnI at 37°C for 30 minutes to digest the vector DNA used as a template, followed by amplification. After agarose gel electrophoresis, a gel portion containing the amplified linear vector DNA fragment is cut out, and the vector DNA fragment is recovered from the gel portion with a GEL/PCR Purification Mini Kit (FAVORGEN).

### (3-2) Preparation of plasmids containing secretory signal peptide genes

The DNA fragments containing secretory signal peptide genes prepared in the section "(3-1) Preparation of secretory signal peptide gene fragments" are separately ligated to the linear pCAMO-6-muGFP DNA fragment using NEBridge Golden Gate Assembly Kit (BsaI-HF v2) manufactured by New England Biolabs. At this time, the restriction enzyme BsaI included in the kit cleaves the sites near the ends of the vector DNA to generate sticky ends, which are then used for ligation by T4 DNA ligase to join the DNAs. This results in the production of a plasmid in which the muGFP-encoding DNA is joined via a 3-nucleotide oligonucleotide to the 3'-end of the secretory signal peptide gene.

*Escherichia coli* strain JM109 is transformed with the resulting reaction mixture by heat shock method, applied to LB medium containing 50 µg/mL kanamycin, and cultured at 37°C for 24 hours.

Strains grown on the LB medium are separately cultured and the respective plasmids are extracted. Their gene sequences are identified as needed. In the case where gene fragments derived from multiple different genes are mixed, a number of different clones grown on LB medium are collected together as needed, and the respective plasmids are extracted to create a secretory signal peptide expression library.

### (4) Transformants of Hydrogenophilus thermoluteolus

### (4-1) Gene transfer and selection

*Hydrogenophilus thermoluteolus* strain TH-1 is transformed with pCAMO-6-muGFP or pCAMO-6-muGFP having an insert DNA fragment containing a secretory signal peptide gene by electric pulse method (electroporation), seeded onto solid LB medium containing 50 µg/mL kanamycin, and cultured at 52°C for 24 hours.

Transformed strains grown on the solid LB medium are separately inoculated with a platinum loop into liquid A medium containing 50 µg/mL kanamycin, and cultured with shaking at 52°C for 24 hours while a gas mixture of H₂:O₂:CO₂ = 7.5:1:1.5 is supplied. The green fluorescent protein in the culture supernatant is quantified at an excitation wavelength of 488 nm and an emission wavelength of 509 nm. Confirmation is made that the fluorescence intensity at 509 nm of the culture supernatant of the transformed strain with the pCAMO-6-muGFP having an insert DNA fragment containing the secretory signal peptide gene (secretory signal peptide gene-containing vector) is higher than that of the culture supernatant of the transformed strain with the pCAMO-6-muGFP not containing the secretory signal peptide gene (control vector).

Here, muGFP is used as the target protein; however, when the target protein cannot be quantified by measuring the fluorescence intensity or absorbance of the culture supernatant, quantification is made using methods such as enzyme activity measurement, SDS-polyacrylamide gel electrophoresis, and western blotting of the culture supernatant.

Through this procedure, the suitability of secretory signal peptides for the secretion of the target protein is evaluated, and a suitable secretory signal peptide for the target protein is selected.

### (4-2) Protein production

A transformed strain with a vector containing a secretory signal peptide gene suitable for a target protein (pCAMO-6 containing a secretory signal peptide gene and the gene of a target protein) selected according to the method described above, and a transformed strain (NC strain) with a control vector (pCAMO-6 containing the gene of the target protein but not the secretory signal peptide gene) are separately inoculated with a platinum loop into liquid A medium containing 50 µg/mL kanamycin, and cultured with shaking at 52°C for 24 hours while a gas mixture of H₂:O₂:CO₂ = 7.5:1:1.5 is supplied. After that, culture supernatants are collected by centrifugation (4°C, 5,000 g, 10 minutes). The culture supernatant of the transformed strain with the vector containing the secretory signal peptide gene suitable for the target protein contains the target protein produced therein, unlike the culture supernatant of the NC strain with the control vector.

*Hydrogenophilus* bacterium transformant and recombinant obtained by introduction of any of the secretory signal peptide genes of the present invention together with the gene of a target protein can be prepared with reference to the description in the specification including the section "EXAMPLES". Furthermore, other bacterial strains described herein are internationally deposited under the Budapest Treaty, maintained by organizations from which any one can unconditionally acquire the strains, commercially available, or can be prepared by those skilled in the art on the basis of this specification. Accordingly, these strains are all available to the general public.

### Industrial Applicability

The DNA of the present invention functions as a secretory signal peptide gene in various types of microorganisms and enables extracellular production of a target protein in an efficient manner. In particular, a transformant or recombinant obtained by introducing, into a *Hydrogenophilus* bacterium, a DNA fragment containing the DNA of the present invention joined to the gene of a target protein in a manner that allows for secretion of the protein can extracellularly produce the target protein using carbon dioxide as its sole carbon source in a highly efficient manner. Therefore, the transformant or recombinant can provide a solution to global warming caused by increased carbon dioxide emissions and achieve highly efficient production of the target protein.

## Claims

1. A DNA of the following (a), (b), or (c).
(a) a DNA encoding a peptide comprising the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151.
(b) a DNA encoding a peptide comprising an amino acid sequence that has 90% or more identity with the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.
(c) a DNA encoding a peptide comprising an amino acid sequence that has 1 to 5 amino acids deleted, substituted, inserted, or added in the amino acid sequence of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, or 151 and functioning as a secretory signal peptide.

2. The DNA according to claim 1 wherein the DNA is a DNA of the following (d) or (e).
(d) a DNA comprising the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152.
(e) a DNA comprising a nucleotide sequence that has 90% or more identity with the nucleotide sequence of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, or 152 and encoding a peptide functioning as a secretory signal peptide.

3. A secretory signal peptide gene comprising the DNA according to claim 1 or 2.

4. A vector comprising the DNA according to claim 1 or 2.

5. A vector for expressing a target protein, the vector comprising the gene of the target protein downstream of the DNA according to claim 1 or 2.

6. A transformant obtained by introducing, into a host microorganism, a DNA fragment containing the DNA according to claim 1 or 2 and the structural gene of a target protein located downstream of the DNA, or the vector according to claim 5.

7. The transformant according to claim 6, wherein the host microorganism is a *Hydrogenophilus* bacterium.

8. A method for producing a target protein, the method comprising the steps of: culturing the transformant according to claim 7 using substantially only carbon dioxide as a carbon source; and collecting the protein from culture medium.

9. A recombinant obtained by introducing the DNA according to claim 1 or 2 into the upstream of the structural gene of a target protein in the genome of a microorganism.

10. The recombinant according to claim 9, wherein the microorganism is a *Hydrogenophilus* bacterium.

11. A method for producing a target protein, the method comprising the steps of: culturing the recombinant according to claim 10 using substantially only carbon dioxide as a carbon source; and collecting the protein from culture medium.
